# EUROPEAN PATENT APPLICATION

(11) **EP 3 281 629 A1**
(43) Date of publication of application: **14.02.2018**
(21) Application number: 16776260.8
(22) Date of filing: 18.03.2016
(51) Int. Cl.: A61K 31/197, A61K 31/295, A61K 31/555, A61K 33/26, A61K 38/16, A61P 31/12, A61P 31/14, A61P 31/20, A61P 43/00

(54) **PROPHYLACTIC/THERAPEUTIC AGENT FOR VIRUS INFECTIONS WHICH COMPRISES ALA COMPOUND**

(30) Priority: 10.04.2015 JP 2015080703
(71) Applicant: SBI Pharmaceuticals Co., Ltd., Tokyo 106-6020 (JP)
(72) Inventor: TANAKA, Tohru, Tokyo 106-6020 (JP); NAKAJIMA, Motowo, Tokyo 106-6020 (JP); HARA, Takeshi, Tokyo 106-6020 (JP)
(74) Representative: EDP Patent Attorneys B.V.
(86) International application number: PCT/JP2016/001609
(87) International publication number: WO 2016/163082

(57) **Abstract**

The present invention addresses the problem of providing a prophylactic/therapeutic agent which is inexpensive, has little adverse side effects and is effective against persistent virus infections. A 5-aminolevulinic acid compound represented by the following formula (I) (wherein R¹ represents a hydrogen atom or an acyl group; and R² represents a hydrogen atom, a linear or branched alkyl group, a cycloalkyl group, an aryl group or an aralkyl group) or a salt thereof can be used as a prophylactic and/or therapeutic agent for virus infections to prevent proliferation of viruses.

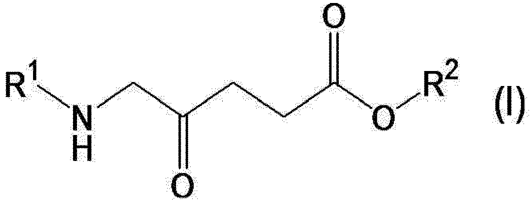

## Description

### Technical Field

The present invention relates to a prophylactic and/or therapeutic agent for a virus infection, and more particularly to a prophylactic and/or therapeutic agent for a virus infection comprising a 5-aminolevulinic acid (5-ALA) or a derivative thereof, or a salt of the 5-aminolevulinic acid or the derivative (also collectively referred to as "ALA compound") and an iron compound.

### Background Art

When a virus enters the body of a host by infection, it is in most cases eliminated from the body in a short time after the infection, for example, by the immune system of the host. However, as contrasted with such a disease caused by a transient infection such as an infection with influenza virus, there are some diseases such as AIDS, hepatitis C, hepatitis B that are caused by viruses which are not eliminated from the body, with which the infection persists a long time and may transition to persistent infection that will in the worst-case scenario cause the host to die.

For example, for hepatitis C, it is considered that after the onset of acute hepatitis in association with the infection with hepatitis C virus (HCV), 30 to 40% of the infected individuals come to have no virus detected and their liver function is normalized, but the remaining 60 to 70% of the infected individuals become HCV carriers and for many cases the acute hepatitis transitions directly to chronic hepatitis. It is also considered that the probability of spontaneous remission from chronic hepatitis is extremely low, i.e., 0.2%, and 10 to 16% of its cases transition to hepatic cirrhosis in the course of an average of 20 years after the initial infection (see for example, Non-patent Document 1).

Hepatitis B a causative virus of which is hepatitis B virus (HBV) may end with a transient infection, but when it becomes chronic, the infection persists a lifetime and may progress to hepatic cirrhosis or liver cancer. Entecavir which is a nucleic acid analog has been heretofore used as a drug for treating hepatitis B which is attributable mainly to the infection with hepatitis B virus. It is considered that entecavir is unsuitable for prolonged administration because it is targeted to reverse transcriptase but it does not directly remove the virus and is prone to develop resistant viruses. It is also considered that entecavir cannot be administrated to women suspected of being pregnant because it may cause teratogenicity (see for example, Non-patent Document 2).

The therapy with interferons which are used as drugs for treating hepatitis B and hepatitis C causes adverse side effects in various ways, and therefore, there is a need for the development of other therapies.

On the other hand, 5-aminolevulinic acid (5-ALA) is known as an intermediate in the tetrapyrrole biosynthesis pathway which is widely found in animals, plants and microorganisms, and is normally produced by biosynthesis from succinyl-CoA and glycine by 5-aminolevulinate synthase. Photodynamic therapy or photokinetic therapy with 5-ALA (ALA-PDT) has been developed and has gained attention as a therapy which is less invasive and can preserve quality of life, and a tumor diagnostic agent with a 5-ALA compound has been reported (see for example, Patent Document 1).

There have been reported a method for treating a virus infection, comprising administering 5-aminolevulinic acid to cause virus-infected cells to accumulate protoporphyrin; and applying a cytocidal dose of red light to the virus-infected, protoporphyrin-accumulated cells to destroy the virus-infected cells (see for example, Patent Document 2); use of 5-ALA hexyl ester, or a pharmaceutically acceptable salt thereof, in the manufacture of a composition for use in photodynamic therapy (PDT) on an animal for the treatment of virus infections of the lining of the vagina, uterine cervix or uterus (see for example, Patent Document 3); and a prophylactic/therapeutic agent for influenza viral infection comprising 5-ALA or a derivative thereof, or a salt of the 5-ALA or the derivative and an iron compound as active ingredients (see for example, Patent Document 4).

### Prior Art Documents

### Patent Documents

Patent Document 1: Japanese Patent No. 2731032
Patent Document 2: Japanese unexamined Patent Application Publication (Translation of PCT Application) No. 2000-510123
Patent Document 3: Japanese unexamined Patent Application Publication No. 2014-94963
Patent Document 4: International Publication No. WO 2014-013664

### Non-patent Documents

Non-patent Document 1: Infectious Diseases Weekly Report Japan of Ministry of Health, Labour and Welfare/National Institute of Infectious Diseases, the 12th week, 2004 (March 15 to 21, 2004) http://idsc.nih.go.jp/idwr/kansen/k04/k04_12.html
Non-patent Document 2: J Infect Dis. (2001) 184 (10): 1236-1245

### Summary of the Invention

### Problem to be Solved by the Invention

Many of conventional therapeutic agents for virus infections are limited to the early stage of infections in the timing of taking effect, have strong adverse side effects, or are expensive. The present invention addresses the problem of providing a prophylactic/therapeutic agent which is inexpensive, has little adverse side effects and is effective against persistent virus infections.

### Means to Solve the Object

There has been previously reported a method for treating virus infections by administering 5-ALA to accumulate protoporphyrin in virus-infected cells and irradiating the virus-infected and protoporphyrin-accumulated cells with light. However, such a method has been severely limited in body sites in which the virus-infected cells can be irradiated with light, and therefore has not been applicable to serious virus infections. The present inventors has long advanced research about 5-ALA in diverse ways. They have now found that, by administering 5-ALA without need for light irradiation, proliferation of viruses in the virus-infected cells can be inhibited and that the effect of inhibiting proliferation of viruses becomes particularly marked 22 days after virus infection. As a result, they have completed the present invention.

That is, the present invention is as follows:
[1] A prophylactic and/or therapeutic agent for a virus infection, comprising a compound represented by the following formula (I): 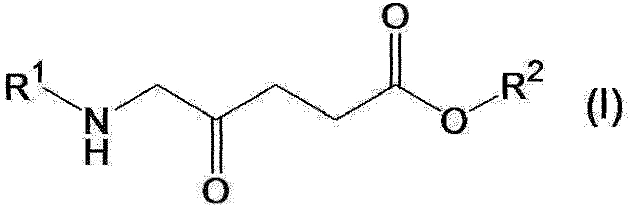 (wherein R¹ represents a hydrogen atom or an acyl group; and R² represents a hydrogen atom, a linear or branched alkyl group, a cycloalkyl group, an aryl group or an aralkyl group)
   or a salt thereof.
[2] The prophylactic and/or therapeutic agent for a virus infection according to [1], wherein R¹ and R² are each a hydrogen atom.
[3] The prophylactic and/or therapeutic agent for a virus infection according to [1] or [2], further comprising an iron compound.
[4] The prophylactic and/or therapeutic agent for a virus infection according to [3], wherein the iron compound is one or more compounds selected from the group consisiting of ferric chloride, iron sesquioxide, iron sulfate, ferrous pyrophosphate, ferrous citrate, iron sodium citrate, sodium ferrous citrate, iron ammonium citrate, ferric pyrophosphate, iron lactate, ferrous gluconate, iron sodium diethylenetriaminepentaacetate, iron ammonium diethylenetriaminepentaacetate, iron sodium ethylenediaminetetraacetate, iron ammonium ethylenediaminetetraacetate, iron sodium dicarboxymethylglutamate, iron ammonium dicarboxymethylglutamate, ferrous fumarate, iron acetate, iron oxalate, ferrous succinate, iron sodium succinate citrate, heme iron, iron dextran, iron triethylenetetramine, lactoferrin iron, transferrin iron, sodium iron chlorophyllin, ferritin iron, saccharated iron oxide and sulfide glycine iron.
[5] The prophylactic and/or therapeutic agent for a virus infection according to [3], wherein the iron compound is sodium ferrous citrate.
[6] The prophylactic and/or therapeutic agent for a virus infection according to any one of [1] to [5], wherein the virus infection is hepatitis B.
[7] The prophylactic and/or therapeutic agent for a virus infection according to any one of [1] to [5], wherein the virus infection is Ebola hemorrhagic fever.
[8] A method for preventing and/or treating a virus infection, comprising administering the prophylactic and/or therapeutic agent according to any one of [1] to [7] to a subject.
[9] A method for preventing and/or treating a virus infection, comprising administering to a subject simultaneously or one after another:
   a) a compound represented by the following formula (I) : 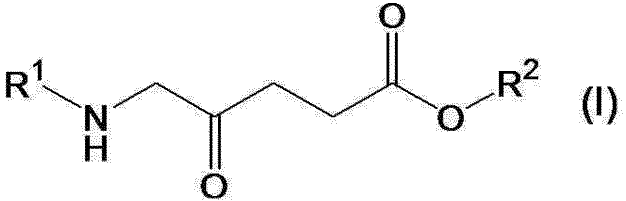 (wherein R¹ represents a hydrogen atom or an acyl group; and R² represents a hydrogen atom, a linear or branched alkyl group, a cycloalkyl group, an aryl group or an aralkyl group) or a salt thereof; and
   b) an iron compound.
[10] The method for preventing and/or treating a virus infection according to [8] or [9], wherein the method does not involve light irradiation.
[11] A kit for preventing and/or treating a virus infection, comprising:
   a) a compound represented by the following formula (I) : 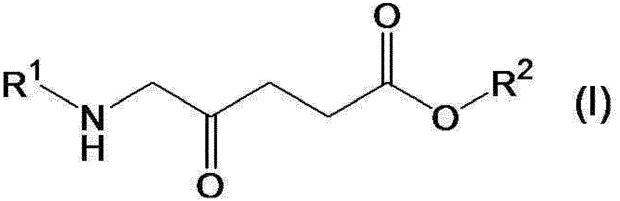 (wherein R¹ represents a hydrogen atom or an acyl group; and R² represents a hydrogen atom, a linear or branched alkyl group, a cycloalkyl group, an aryl group or an aralkyl group)
      or a salt thereof; and
   b) an iron compound.
[12] A combination of prophylactic and/or therapeutic agents, comprising:
   a) the prophylactic and/or therapeutic agent for a virus infection according to any one of "1" to "7"; and
   b) an antiviral infection agent.
[13] A combination of prophylactic and/or therapeutic agents, comprising:
   a) a compound represented by the following formula (I) : 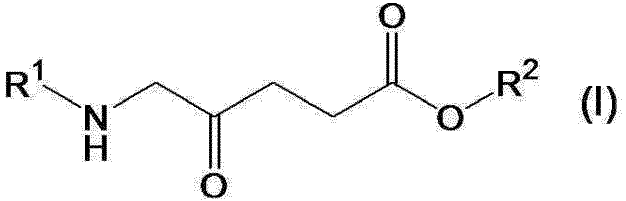 (wherein R¹ represents a hydrogen atom or an acyl group; and R² represents a hydrogen atom, a linear or branched alkyl group, a cycloalkyl group, an aryl group or an aralkyl group)
      or a salt thereof;
   b) an iron compound; and
   c) an antiviral infection agent.
[14] A compound represented by the following formula (I) : 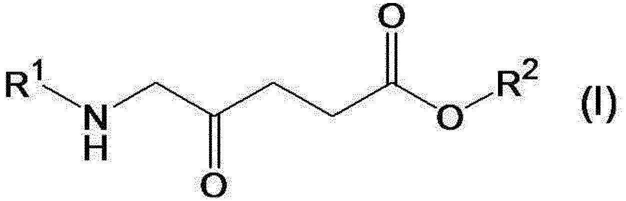 (wherein R¹ represents a hydrogen atom or an acyl group; and R² represents a hydrogen atom, a linear or branched alkyl group, a cycloalkyl group, an aryl group or an aralkyl group) or a salt thereof for use as an antiviral infection agent.
[15] A compound represented by the following formula (I) : 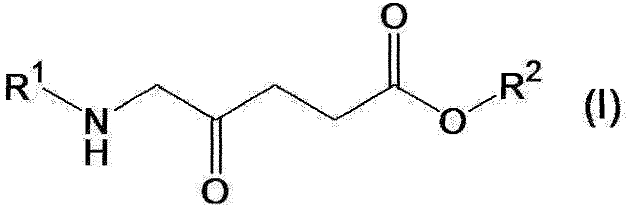 (wherein R¹ represents a hydrogen atom or an acyl group; and R² represents a hydrogen atom, a linear or branched alkyl group, a cycloalkyl group, an aryl group or an aralkyl group)
   or a salt thereof and an iron compound for use as an antiviral infection agent.
[16] Use of a compound represented by the following formula (I)

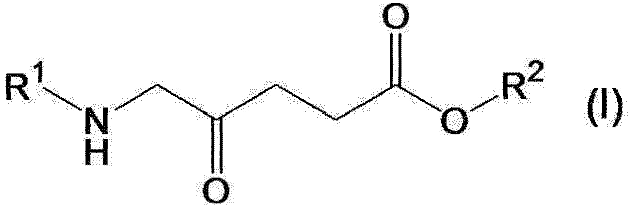

   (wherein R¹ represents a hydrogen atom or an acyl group; and R² represents a hydrogen atom, a linear or branched alkyl group, a cycloalkyl group, an aryl group or an aralkyl group)
   or a salt thereof; b) an iron compound, for preparing an antiviral infection agent.
[17] Use of a compound represented by the following formula (I) 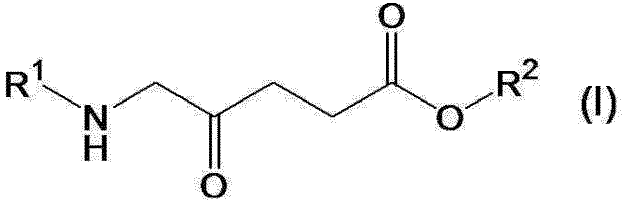 (wherein R¹ represents a hydrogen atom or an acyl group; and R² represents a hydrogen atom, a linear or branched alkyl group, a cycloalkyl group, an aryl group or an aralkyl group) or a salt thereof and an iron compound for preparing an antiviral infection agent.

### Effect of the Invention

The present invention can provide a prophylactic and/or therapeutic agent for virus infections which can be used for preventing and/or treating virus infections, which can avoid virus infections from becoming severe to thereby improve the survival rate, and which has little adverse side effects.

### Brief Description of Drawings

[Figure 1] Figure 1 is a graph showing the effect of inhibiting HBV by the prophylactic and/or therapeutic agent for a virus infection according to the present invention. The ordinate indicates the DNA content of HBV in the culture supernatants. Five log copies/mL corresponds to 1 × 10⁵ viruses/mL.
[Figure 2] Figure 2 is a graph showing the measurement values of ALT and AST when administering to a human being the prophylactic and/or therapeutic agent for a virus infection according to the present invention.
[Figure 3] Figure 3 is a graph showing the measurement values of total bilirubin when administering to human the prophylactic and/or therapeutic agent for a virus infection according to the present invention.

### Mode of Carrying Out the Invention

The prophylactic and/or therapeutic agent for a virus infection according to the present invention is not particularly limited, as long as it comprises a compound represented by the above formula (I) or a salt thereof. It preferably comprises an iron compound in addition to the ALA compound. Examples of the treatment or prevention of a virus infection in the present invention include decreasing viral counts or eliminating viruses in living body, preventing or inhibiting the development of symptoms caused by virus infections, and alleviating such symptoms.

The virus infections in the present invention are not particularly limited, as long as they are diseases or diseases exhibiting symptoms caused by viruses that are infectious microorganisms which enter a living cell and proliferate (replicate) therein. Examples of the virus causing the disease include a virus causing a virus disease such as hepatitis B virus, hepatitis C virus, Ebola virus, AIDS virus, herpes simplex virus, varicellazoster virus and smallpox virus.

The kit for preventing and/or treating a virus infection according to the present invention are not particularly limited, as long as it comprises an ALA compound or an iron compound, separately. The method for preventing and/or treating a virus infection according to the present invention with the kit for preventing and/or treating a virus infection according to the present invention is characterized by administering the ALA compound and the iron compound to a subject simultaneously or one after another without need for light irradiation.

The combination of prophylactic and/or therapeutic agents according to the present invention is not particularly limited, as long as it is the combination of prophylactic and/or therapeutic agents comprising the above-mentioned prophylactic and/or therapeutic agent for a virus infection according to the present invention and an antiviral infection agent, or the combination of prophylactic and/or therapeutic agents comprising an ALA compound, an iron compound and an antiviral infection agent. Such a combination of prophylactic and/or therapeutic agents can be administered to prevent and/or treat a virus infection. Each of the preparations (ingredients) of the combination can be administered simultaneously or separately.

Among the above ALA compounds, the 5-ALA represented by the formula (I) wherein R¹ and R² are each a hydrogen atom is an amino acid also referred to as δ-aminolevulinic acid. Examples of the derivative of the 5-ALA include compounds other than 5-ALA represented by the formula (I) wherein R¹ is a hydrogen atom or an acyl group, and R² is a hydrogen atom, a linear or branched alkyl group, a cycloalkyl group, an aryl group or an aralkyl group.

Examples of the acyl group in the formula (I) include a linear or branched alkanoyl group having 1 to 8 carbon atoms such as formyl group, acetyl group, propionyl group, butyryl group, isobutyryl group, valeryl group, isovaleryl group, pivaloyl group, hexanoyl group, octanoyl group and benzylcarbonyl group; and an aroyl group having 7 to 14 carbon atoms such as benzoyl group, 1-naphthoyl group and 2-naphthoyl group.

Examples of the alkyl group in the formula (I) include a linear or branched alkyl group having 1 to 8 carbon atoms such as methyl group, ethyl group, propyl group, isopropyl group, butyl group, isobutyl group, sec-butyl group, tert-butyl group, pentyl group, isopentyl group, neopentyl group, hexyl group, heptyl group and octyl group.

Examples of the cycloalkyl group in the formula (I) include a cycloalkyl group having 3 to 8 carbon atoms which may contain a saturated bond or a partially unsaturated bond such as cyclopropyl group, cyclobutyl group, cyclopentyl group, cyclohexyl group, cycloheptyl group, cyclooctyl group, cyclododecyl group and 1-cyclohexenyl group.

Examples of the aryl group in the formula (I) include an aryl group having 6 to 14 carbon atoms such as phenyl group, naphthyl group, anthryl group and phenanthryl group.

Examples of the aralkyl group in the formula (I) include a group which contains as an aryl moiety the same group as the above-mentioned aryl group and as an alkyl moiety the same group as the above-mentioned alkyl group, specifically an aralkyl group having 7 to 15 carbon atoms such as benzyl group, phenethyl group, phenylpropyl group, phenylbutyl group, benzhydryl group, trityl group, naphthylmethyl group and naphthylethyl group.

The above-mentioned ALA compounds may be any compound which exerts its effect in the form of 5-ALA represented by the formula (I) or a derivative thereof, and can be administered, depending on the dosage form thereof, as an ALA salt having the solubility enhanced, or as a prodrug (precursor) such as an ester derivative of 5-ALA which is degraded by an in vivo enzyme. Examples of the salt of 5-ALA and the derivative thereof include a pharmacologically acceptable acid addition salt, a pharmacologically acceptable metal salt, a pharmacologically acceptable ammonium salt and a pharmacologically acceptable organic amine addition salt. Examples of the acid addition salt include an inorganic acid salt such as a hydrochloride, a hydrobromide, a hydroiodide, a phosphate, a nitrate and a sulfate, and an organic acid addition salt such as a formate, an acetate, a propionate, a toluenesulfonate, a succinate, an oxalate, a lactate, a tartrate, a glycolate, a methanesulfonate, a butyrate, a valerate, a citrate, a fumarate, a maleate and a malate. Examples of the metal salt include an alkali metal salt such as a lithium salt, a sodium salt and a potassium salt, an alkaline-earth metal salt such as a magnesium salt and a calcium salt, and a metal salt such as an aluminum salt and a zinc salt. Examples of the ammonium salt include an ammonium salt and an alkylammonium salt such as a tetramethylammonium salt. Examples of the organic amine salt include a salt such as a triethylamine salt, a piperidine salt, a morpholine salt and a toluidine salt. These salts can be used by dissolving it before use.

Particularly desirable examples of the above-mentioned ALA compound include the above-mentioned 5-ALA, and an ester derivative of the ALA such as ALA methyl ester, ALA ethyl ester, ALA propyl ester, ALA butyl ester and ALA pentyl ester, and a hydrochloride, a phosphate and a sulfate thereof, and particularly preferably ALA hydrochloride and ALA phosphate.

The above-mentioned ALA compounds can be produced by any known method of chemical synthesis, production by microorganisms or production by enzymes. The above-mentioned ALA compounds may be in the form of hydrates or solvates thereof, or can be used alone or in combination of two or more thereof.

Examples of the above-mentioned iron compound, which may be an organic salt or an inorganic salt, include an inorganic salt including ferric chloride, iron sesquioxide, iron sulfate and ferrous pyrophosphate; and an organic salt including an organic acid salt such as carboxylate, for example, a hydroxycarboxylate such as a citrate, e.g. ferrous citrate, iron sodium citrate, sodium ferrous citrate and iron ammonium citrate, ferric pyrophosphate, iron lactate, ferrous gluconate, iron sodium diethylenetriaminepentaacetate, iron ammonium diethylenetriaminepentaacetate, iron sodium ethylenediaminetetraacetate, iron ammonium ethylenediaminetetraacetate, iron sodium dicarboxymethylglutamate, iron ammonium dicarboxymethylglutamate, ferrous fumarate, iron acetate, iron oxalate, ferrous succinate, iron and sodium succinate citrate, and heme iron, iron dextran, iron triethylenetetramine, lactoferrin iron, transferrin iron, sodium iron chlorophyllin, ferritin iron, saccharated iron oxide, and sulfide glycine iron. One or more of such iron compounds can be used.

The molar dosage of the above-mentioned iron compound may be 0 to 100 times the molar dosage of the ALA compound, and is preferably 0.01 to 10 times and more preferably 0.1 to 8 times.

The ALA compound and the iron compound contained in the prophylactic and/or therapeutic agent for a virus infection according to the present invention can be administered as a composition containing both the ALA compound and the iron compound, or alone separately, and preferably alone separately but simultaneously. As used herein, "simultaneously" means that the administration is carried out not only at the same time but also at different times with no significant interval between the administration of the ALA compound and the iron compound so that the administration of both compounds exhibits an additive effect and preferably a synergistic effect.

Examples of the administration route of the prophylactic and/or therapeutic agent for a virus infection according to the present invention include an oral administration including a sublingual administration; or alternatively a parenteral administration such as a direct administration into the kidney by catheter; an inhalation administration; an intravenous administration including an intravenous infusion; a transdermal administration, for example, with a patch; a suppository; or administration by forced enteral nutrition with a nasogastric tube, a nasointestinal tube, a gastrostomy tube or an enterostomy tube.

The prophylactic and/or therapeutic agent for a virus infection according to the present invention can be used in food applications such as specified health foods, health-promoting foods and supplements, in addition to pharmaceutical agents. Examples of the dosage form of the prophylactic and/or therapeutic agent for a virus infection according to the present invention, which can be determined appropriately depending on the above-mentioned administration route, include an injection, an infusion, a tablet, a capsule, a subtle granule, a powder, a solution, a liquor dissolved, for example, in a syrup, a patch and a suppository.

In preparing the prophylactic and/or therapeutic agent for a virus infection according to the present invention, it is possible to add, if necessary, a pharmacologically acceptable carrier, excipient, diluent, additive, disintegrator, binder, coating agent, lubricator, gliding agent, lubricant, flavorant, sweetening agent, solubilizing agent, solvent, gelling agent, nutrient and the like, such as water, physiological saline, animal fat and oil, vegetable oil, lactose, starch, gelatin, crystalline cellulose, gum, talc, magnesium stearate, hydroxypropylcellulose, polyalkylene glycol, polyvinyl alcohol and glycerin. When preparing the prophylactic and/or therapeutic agent for a virus infection according to the present invention as an aqueous solution, it is preferable to pay attention so that the aqueous solution does not become alkaline to prevent decomposition of the ALA compound. When the aqueous solution becomes alkaline, it is also possible to prevent decomposition by removing oxygen.

The dosage and frequency and period of administration of the prophylactic and/or therapeutic agent for a virus infection according to the present invention vary depending on the age, body weight, symptom and the like of a virus-infected patient.
The dosage of the ALA compound can be 0.01 mmol to 25 mmol/day, preferably 0.025 mmol to 7.5 mmol/day, more preferably 0.075 mmol to 5.5 mmol/day, and further more preferably 0.2 mmol to 2 mmol/day, in terms of the molar amount of 5-ALA per adult. Examples of the frequency of administration can include single or multiple daily dosing or continuous administration by intravenous infusion and the like. The period of administration can be determined based on an indicator of virus infections by any method known in the art by pharmacologists or clinicians.

Examples of the aspect substantially equivalent to the present invention include use of the ALA compound in preparing the prophylactic and/or therapeutic agent for a virus infection according to the present invention, and use of the ALA compound in preventing and/or treating a virus infection without photodynamic therapy according to the present invention. The contents of these uses and methods or their preferable aspects are as described above.

The present invention will be now described in detail by examples below but is in no way to be limited thereto.

### Examples

### Example 1

### (Test cells)

PXB hepatocytes prepared from "PXB-Mouse (registered trademark)" (manufactured by PhoenixBio) were used as test samples to examine the effect of a change in virus load by adding 5-ALA or the like to a medium. As in PXB hepatocytes 70% or more of the mouse hepatocytes are replaced by normal human hepatocytes, the liver of PXB-Mouse exhibits metabolism close to the liver of human and becomes infected with human hepatitis B virus and human hepatitis C virus. Therefore, PXB-Mouse is utilized as a human in vivo prediction model of hepatitis virus infections in pharmacokinetic studies.

### (Preparation of the HBV infection medium)

Hepatitis B virus (HBV) genotype C was suspended in dHCGM medium (DMEM + 10% FBS, 44 mM NaHCO₃, 15 µg/mL L-proline, 0.25 µg/mL insulin, 50 µM dexamethasone, 5 ng/mL EGF, 0.1 mM Asc-2P, 2% DMSO) containing 4% polyethylene glycol in the virus load of 2 × 10⁶ per well to give 250 µL/well of a HBV infection medium. The sequence of HBV genotype C is disclosed in a public data bank such as GenBank and the accession number is AB246345.1.

### (Preparation of the test media)

5-ALA and sodium ferrous citrate (SFC) were blended in dHCGM medium at the concentrations shown in Table 1 below to prepare 250 µL each of test media. As a positive control medium, a medium prepared by dissolving 2.5 nM entecavir (manufactured by Santa Cruz Biotechnology, Inc.) in dHCGM medium was used.

**[Table 1]**

| Test medium | 5-ALA (*µ*M) | SFC (*µ*M) | Entecavir (nM) |
|---|---|---|---|
| Negative control (Infection medium) | 0 | 0 | 0 |
| ALA/S FC 100/50*µ*M | 100 | 50 | 0 |
| AL A/S F C 300/150 *µ*M | 300 | 150 | 0 |
| ALA/S F C 1000/500 *µ*M | 1000 | 500 | 0 |
| ALA 1000 *µ*M | 1000 | 0 | 0 |
| SFC 500 *µ* M | 0 | 500 | 0 |
| Entecavir (Positive control) | 0 | 0 | 2. 5 |
| Non-infection medium | 0 | 0 | 0 |

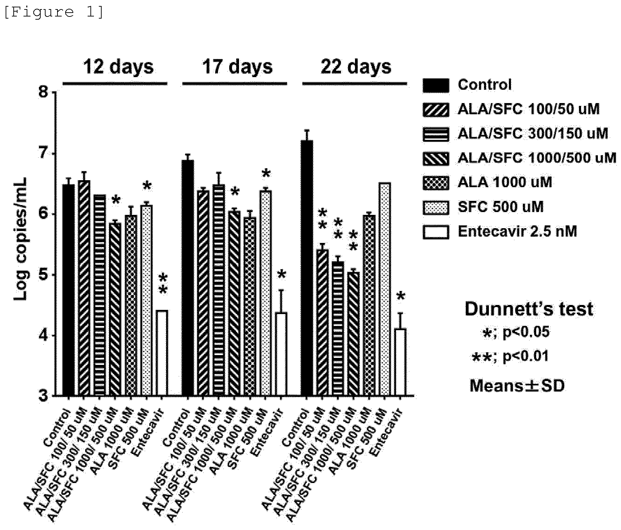

PXB hepatocytes, which are primary cultured liver cells isolated by a two-step collagenase perfusion procedure (Yamasaki C, et al. Drug Metab Pharmacokinet. 2010, 25:539-550.) from 12- to 16-week-old PXB mice having a body weight of 11 g or more, were suspended in Dulbecco's modified Eagle's medium (DMEM) with 10% fetal bovine serum (FBS) (DMEM + 10% FBS) containing 100 IU/mL penicillin G, 100 µg/mL streptomycin and 20 mM HEPES, seeded in a 24-well plate coated with type I collagen at a density of 4 × 10⁵ cells/well, and incubated at 37°C under 5% CO₂. The next day, the medium was removed, and 500 µL of DMEM + 10% FBS was then added to each well. Thereafter, the medium was removed again, 250 µL of the above-mentioned HBV infection medium or a non-infection medium and 250 µL each of the above-mentioned test medium were added to each well to provide 500 µL each of evaluation media. The evaluation media were incubated at 37°C under 5% CO₂ for 20 to 28 hours. On day 1, 2, 7, 12 and 17 after HBV virus infection, the media were replaced by 500 µL each of the above-mentioned evaluation media. On day 12, 17 and 22 after the virus infection, culture supernatants were collected and the DNA content of HBV in the supernatants was determined by a quantitative PCR assay with TaqMan probe. The incubation period after the virus infection was 22 days. The results are shown in the following Tables 2 and 3 and the graph of Figure 1. The ordinate of Figure 1 indicates the DNA content of HBV in the culture supernatants.

### (Results)

As is clearly shown in Figure 1, on day 12 and 17 after the virus infection, proliferation of HBV virus was confirmed to be significantly inhibited at "ALA/SFC 1000/500 µM" and "SFC 500 µM" (*; P < 0.05), and on day 22 after the virus infection, proliferation of HBV virus was confirmed to be remarkably significantly inhibited at "ALA/SFC 100/50 µM", "ALA/SFC 300/150 µM" and "ALA/SFC 1000/500 µM" (**; P < 0.01). The in the culture supernatants of uninfected cells was below the detection limit (see Table 3).

**[Table 2]**

| **Com.** | **Conc.** | **No.** | **day 12** | **day 17** | **day 22** |
|---|---|---|---|---|---|
| **ALA** | 1 mM | 1 | 6.0 | 6.0 | 6.1 |
| | | 2 | 6.1 | 6.0 | 5.9 |
| | | 3 | 5.8 | 5.8 | 6.0 |
| **SFC** | 0.5 mM | 1 | 6.1 | 6.3 | 6.5 |
| | | 2 | 6.1 | 6.4 | 6.5 |
| | | 3 | 6.2 | 6.4 | 6.5 |
| **ALA/SFC** | 1/0.5 mM | 1 | 5.9 | 6.0 | 5.0 |
| | | 2 | 5.8 | 6.1 | 5.0 |
| | | 3 | 5.8 | 6.0 | 5.1 |
| | 0.3/0.15 mM | 1 | 6.3 | 6.3 | 5.1 |
| | | 2 | 6.3 | 6.4 | 5.2 |
| | | 3 | 6.3 | 6.7 | 5.3 |
| | 0.1/0.05 mM | 1 | 6.7 | 6.4 | 5.3 |
| | | 2 | 6.4 | 6.4 | 5.4 |
| | | 3 | 6.5 | 6.3 | 5.5 |
| **ETV** | 2.5 nM | 1 | 4.4 | 4.2 | 4.4 |
| | | 2 | 4.4 | 4.8 | 4.0 |
| | | 3 | 4.4 | 4.1 | 3.9 |
| Untreated (Negative control) | | 1 | 6.4 | 6.8 | 7.0 |
| | | 2 | 6.4 | 6.8 | 7.3 |
| | | 3 | 6.6 | 7.0 | 7.3 |

**[Table 3]**

| **Com.** | **No.** | **day 12** | **day 17** | **day 22** |
|---|---|---|---|---|
| Uninfected | 1 | <3.1 | <3.1 | <3.1 |
| | 2 | <3.1 | <3.1 | <3.1 |
| | 3 | <3.1 | <3.1 | <3.1 |

### Example 2

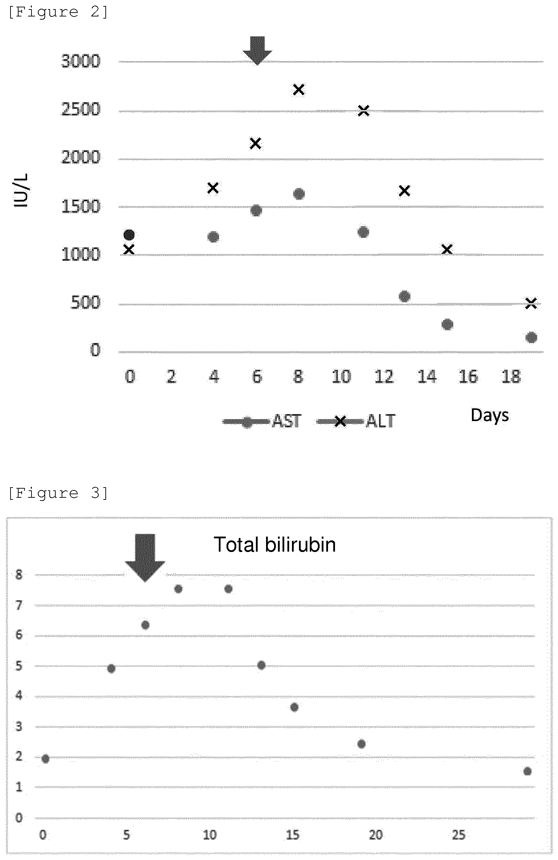

A capsule containing 220 mg of ALA phosphate and 63 mg of sodium ferrous citrate (SFC) (ALA-SFC) was started to be orally administered once daily to a 54 year-old male patient suffering from hepatitis B from day 6 after admission (provided that the date of admission is day 0 of admission) which was taken as day 0 of administration. After the administration was started, the levels of aspartate aminotransferase (AST) and alanine aminotransferase (ALT) in blood, which are predicted to rise as the destruction of hepatocytes proceeds, and the level of total bilirubin in blood, including conjugated bilirubin, which is believed to leak from bile into blood due to hepatic disorder, and unconjugated bilirubin, the level of which is predicted to rise due to the destruction of erythrocytes, are measured. The results are shown in Figure 2 and Figure 3.

### (Results)

As is clearly shown in Figure 2, both levels of AST and ALT increased until day 2 of ALA-SFC administration (day 8 after admission) and ALT and AST reached 2734 IU/L and 1646 IU/L, respectively. Thereafter, they declined and on day 13 of administration (day 19 after admission), ALT and AST were 512 IU/L and 145 IU/L, respectively. Although data are not shown, on day 23 of administration (day 29 after admission), ALT reached 126 IU/L which is 0.046 times its maximum level, and AST reached 59 IU/L which is 0.036 times its maximum level, confirming that both of ALT and AST levels were remarkably improved by ALA-SFC administration.

As is clearly shown in Figure 3, the level of total bilirubin increased until day 5 of ALA-SFC administration (day 11 after admission) and reached 7.6 mg/dL. Thereafter, it declined and on day 13 of administration (day 19 after admission) decreased to 2.5 mg/dL. Although data are not shown, on day 23 of administration (day 29 after admission), the level of total bilirubin reached 1.6 mg/dL which is 0.21 times its maximum level, confirming that the level of total bilirubin was remarkably improved by ALA-SFC administration.

In addition, ten months after the start of ALA-SFC administration, it was confirmed by PCR assay that no HBV virus was detected in blood of the above-mentioned patient. Therefore, it was confirmed that proliferation of the HBV viruses was remarkably inhibited and the viruses were eliminated by ALA-SFC administration.

### Industrial Applicability

The prophylactic and/or therapeutic agent for a virus infection according to the present invention is useful in the medical field.

## Claims

1. A prophylactic and/or therapeutic agent for a virus infection, comprising a compound represented by the following formula (I): 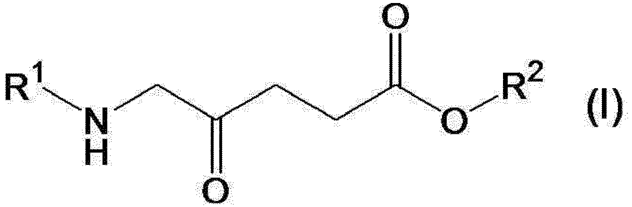 (wherein R¹ represents a hydrogen atom or an acyl group; and R² represents a hydrogen atom, a linear or branched alkyl group, a cycloalkyl group, an aryl group or an aralkyl group)
or a salt thereof.

2. The prophylactic and/or therapeutic agent for a virus infection according to claim 1, wherein R¹ and R² are each a hydrogen atom.

3. The prophylactic and/or therapeutic agent for a virus infection according to claim 1 or 2, further comprising an iron compound.

4. The prophylactic and/or therapeutic agent for a virus infection according to claim 3, wherein the iron compound is one or more compounds selected from the group consisting of ferric chloride, iron sesquioxide, iron sulfate, ferrous pyrophosphate, ferrous citrate, iron sodium citrate, sodium ferrous citrate, iron ammonium citrate, ferric pyrophosphate, iron lactate, ferrous gluconate, iron sodium diethylenetriaminepentaacetate, iron ammonium diethylenetriaminepentaacetate, iron sodium ethylenediaminetetraacetate, iron ammonium ethylenediaminetetraacetate, iron sodium dicarboxymethylglutamate, iron ammonium dicarboxymethylglutamate, ferrous fumarate, iron acetate, iron oxalate, ferrous succinate, iron sodium succinate citrate, heme iron, iron dextran, iron triethylenetetramine, lactoferrin iron, transferrin iron, sodium iron chlorophyllin, ferritin iron, saccharated iron oxide and sulfide glycine iron.

5. The prophylactic and/or therapeutic agent for a virus infection according to claim 3, wherein the iron compound is sodium ferrous citrate.

6. The prophylactic and/or therapeutic agent for a virus infection according to any one of claims 1 to 5, wherein the virus infection is hepatitis B.

7. The prophylactic and/or therapeutic agent for a virus infection according to any one of claims 1 to 5, wherein the virus infection is Ebola hemorrhagic fever.

8. A method for preventing and/or treating a virus infection, comprising administering the prophylactic and/or therapeutic agent according to any one of claims 1 to 7 to a subject.

9. A method for preventing and/or treating a virus infection, comprising administering to a subject simultaneously or one after another:
a) a compound represented by the following formula (I): 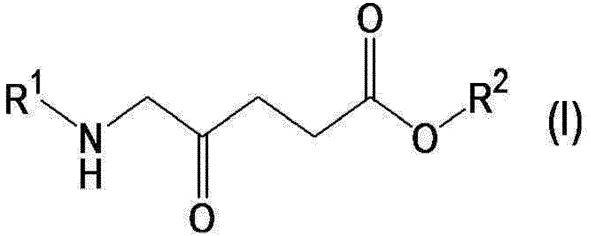 (wherein R¹ represents a hydrogen atom or an acyl group; and R² represents a hydrogen atom, a linear or branched alkyl group, a cycloalkyl group, an aryl group or an aralkyl group)
or a salt thereof; and
b) an iron compound.

10. The method for preventing and/or treating a virus infection according to claim 8 or 9, wherein the method does not involve light irradiation.

11. A kit for preventing and/or treating a virus infection, comprising:
a) a compound represented by the following formula (I): 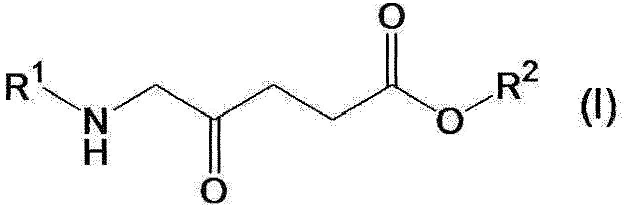 (wherein R¹ represents a hydrogen atom or an acyl group; and R² represents a hydrogen atom, a linear or branched alkyl group, a cycloalkyl group, an aryl group or an aralkyl group)
or a salt thereof; and
b) an iron compound.

12. A combination of prophylactic and/or therapeutic agents, comprising:
a) the prophylactic and/or therapeutic agent for a virus infection according to any one of claims 1 to 7; and
b) an antiviral infection agent.

13. A combination of prophylactic and/or therapeutic agents, comprising:
a) a compound represented by the following formula (I): 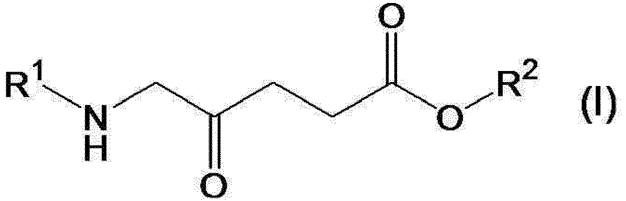 (wherein R¹ represents a hydrogen atom or an acyl group; and R² represents a hydrogen atom, a linear or branched alkyl group, a cycloalkyl group, an aryl group or an aralkyl group)
or a salt thereof;
b) an iron compound; and
c) an antiviral infection agent.

14. A compound represented by the following formula (I): 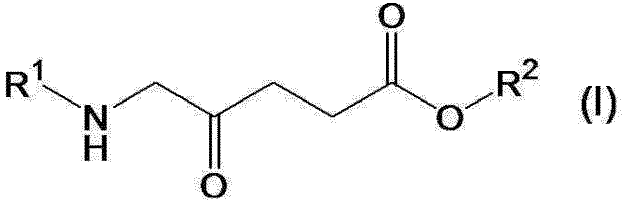 (wherein R¹ represents a hydrogen atom or an acyl group; and R² represents a hydrogen atom, a linear or branched alkyl group, a cycloalkyl group, an aryl group or an aralkyl group)
or a salt thereof for use as an antiviral infection agent.

15. A compound represented by the following formula (I): 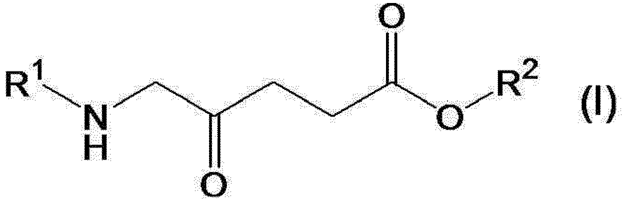 (wherein R¹ represents a hydrogen atom or an acyl group; and R² represents a hydrogen atom, a linear or branched alkyl group, a cycloalkyl group, an aryl group or an aralkyl group)
or a salt thereof and an iron compound for use as an antiviral infection agent.

16. Use of a compound represented by the following formula (I) 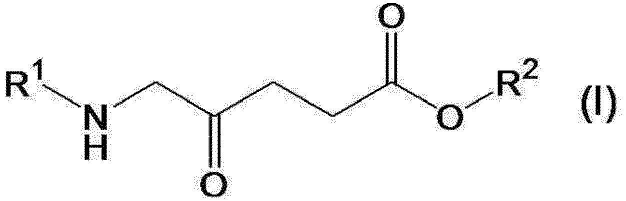 (wherein R¹ represents a hydrogen atom or an acyl group; and R² represents a hydrogen atom, a linear or branched alkyl group, a cycloalkyl group, an aryl group or an aralkyl group)
or a salt thereof;
b) an iron compound,
for preparing an antiviral infection agent.

17. Use of a compound represented by the following formula (I) 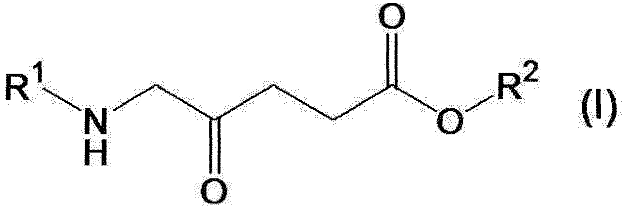 (wherein R¹ represents a hydrogen atom or an acyl group; and R² represents a hydrogen atom, a linear or branched alkyl group, a cycloalkyl group, an aryl group or an aralkyl group)
or a salt thereof and an iron compound for preparing an antiviral infection agent.
